Europäisches Patentamt

European Patent Office

Office européen des brevets

(11) Veröffentlichungsnummer : **0 080 037 B1**

(12) **EUROPÄISCHE PATENTSCHRIFT**

(45) Veröffentlichungstag der Patentschrift :
26.06.85

(21) Anmeldenummer : 82108637.8

(22) Anmeldetag : 18.09.82

(51) Int. Cl.⁴ : **A 61 M 5/20**, A 61 B 6/00,
G 01 F 13/00, A 61 M 5/14

(54) Injektionsvorrichtung zur dosierten Abgabe einer Flüssigkeit.

(30) Priorität : 24.11.81 CH 7500/81

(43) Veröffentlichungstag der Anmeldung :
01.06.83 Patentblatt 83/22

(45) Bekanntmachung des Hinweises auf die Patenterteilung : 26.06.85 Patentblatt 85/26

(84) Benannte Vertragsstaaten :
CH DE FR GB IT LI NL SE

(56) Entgegenhaltungen :
FR-A- 2 171 971
GB-A- 1 165 842
US-A- 3 504 777

(73) Patentinhaber : CONTRAVES AG
Schaffhauserstrasse 580
CH-8052 Zürich (CH)

(72) Erfinder : Rüegg, André
Ueberlandstrasse 451
CH-8051 Zürich (CH)

## Beschreibung

Die Erfindung bezieht sich auf eine Injektionsvorrichtung zur dosierten Abgabe einer Flüssigkeit, bestehend aus einem Injektorgehäuse, einem am vorderen Ende des Injektorgehäuses angeordneten Halterungskörper und einer in dem Halterungskörper auswechselbar angeordneten Injektionsspritze, welche im wesentlichen einen Zylinderkörper mit einem Flansch an dem dem Halterungskörper zugewandten Ende und einen im Zylinderkörper geführten Kolben umfasst, der durch eine Kolbenstange mit der motorisch angetriebenen Spindelwelle einer in dem Injektorgehäuse angeordneten Spindelmechanik wirkverbunden ist.

Aus der DE-C-28 05 513 ist eine Vorrichtung zur dosierten Abgabe einer Flüssigkeit bekannt, welche im wesentlichen einen in einem Injektorgehäuse angeordneten, motorisch angetriebenen Spindelmechanismus, eine in eine Halterung eingesetzte Injektionsspritze und einen im Zylinderkörper der Injektionsspritze geführten Kolben aufweist, der durch eine Kolbenstange mit der Spindelwelle verbunden ist. An der Spindelwelle ist eine erste Skalen-Trommel sowie eine mit der ersten Skalen-Trommel lösbar gekuppelte zweite Skalen-Trommel angeordnet, wobei die Skalen-Trommeln für die Begrenzung der Volumen-Einstellung sowie für die Begrenzung der Vorschubbewegung ausgebildet sind. Mit dieser Vorrichtung wird ein exakter Steuerungsablauf und somit eine für den Patienten lebenswichtige, exakt dem vom Arzt eingestellten Volumen entsprechende Injizierung gewährleistet.

Der Erfindung liegt die Aufgabe zugrunde, für eine Injektionsvorrichtung gemäss der im Oberbegriff des Anspruchs 1 genannten Art eine Einrichtung zu schaffen, mit deren Hilfe Injectionsspritzen mit unterschiedlichem Zylinderquerschnitt beim Einsetzen in den Halterungskörper abgetastet und in Abhängigkeit vom Zylinderquerschnitt die Vorschubgeschwindigkeit des in dem Zylinderkörper geführten Kolbens einstellbar ist.

Gemäss der Erfindung wird diese Aufgabe dadurch gelöst, dass in dem Halterungskörper mindestens ein Tastbügel auslenkbar gelagert ist, welcher auf der einen Seite ein den Flansch des Zylinderkörpers kontaktierendes Tastteil und auf der anderen Seite im Injektorteil ein Betätigungsteil aufweist, welchem mindestens ein Schalter zugeordnet ist, der gewährleistet, dass bei ausgelenktem Tastbügel zur Einstellung der Vorschubgeschwindigkeit des mit der Kolbenstange wirkverbundenen Kolbens ein vom Zylinderquerschnitt abhängiges Signal auf die Spindelmechanik übertragen wird.

Weitere Merkmale der Erfindung ergeben sich aus der folgenden Beschreibung in Verbindung mit der Zeichnung und den Patentansprüchen.

Die Erfindung wird nachstehend unter bezugnahme auf die Zeichnung näher beschrieben. Es zeigt :

Figur 1 eine in Ansicht dargestellte und an einem Ständer befestigte Injektionsvorrichtung,

Figur 2 einen in grösserem Massstab und teilweise im Schnitt dargestellten Ausschnitt der in Fig. 1 durch einen Kreis bezeichneten Stelle Y mit dem mit einer Abtasteinrichtung versehenen Halterungskörper für eine erste Injektionsspritze,

Figur 3 ein in Ansicht dargestelltes Teilstück einer zweiten Injektionsspritze,

Figur 4 eine im Schnitt und in Draufsicht dargestellte erste Hälfte des Halterungskörpers mit der eingesetzten ersten Injektionsspritze gemäss Fig. 2, und

Figur 5 die im Schnitt und in Draufsicht dargestellte zweite Hälfte des Halterungskörpers mit der eingesetzten zweiten Injektionsspritze gemäss Fig. 3.

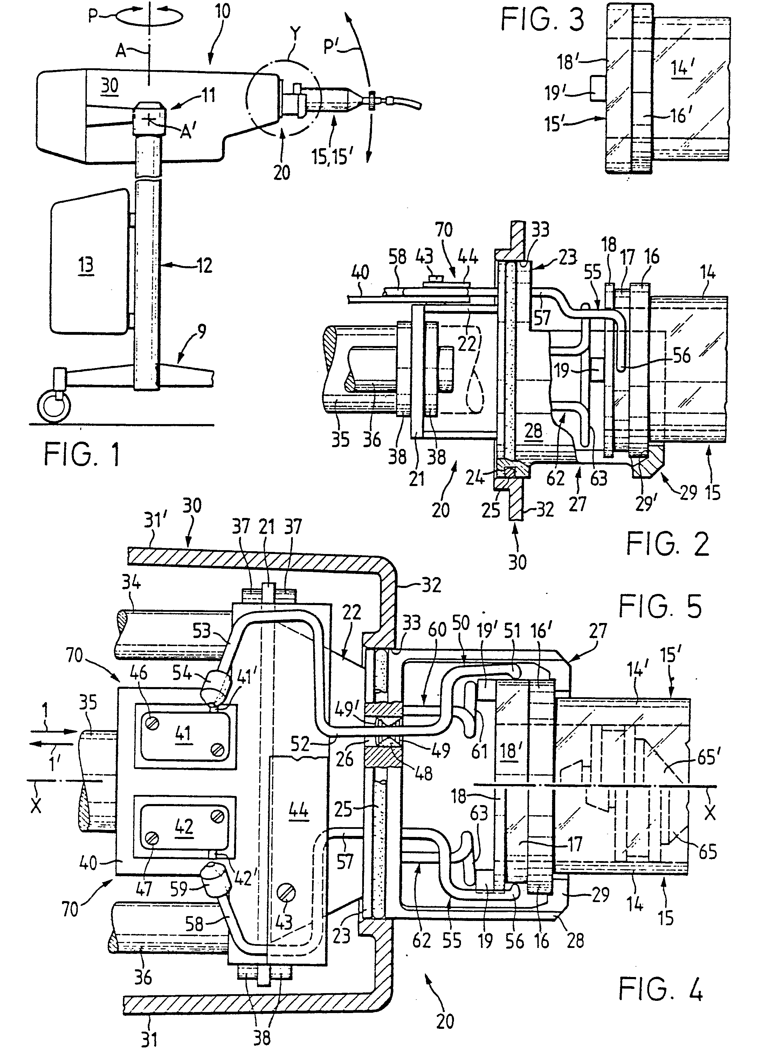

In Fig. 1 ist in Ansicht ein medizinisches Gerät, insbesondere eine Injektionsvorrichtung 10 schematisch dargestellt, welche im wesentlichen ein auf Rollen gelagertes Fussgestell 9, einen Ständer 12, einen am oberen Ende des Ständers angeordneten Schwenkkopf 11 sowie ein daran mit nicht dargestellten Mitteln befestigtes Injektorgehäuse 30 umfasst. Seitlich an dem Ständer 12 ist ein Steuer- und Bedienungspult 13 angeordnet und befestigt, welches über nicht dargestellte Kabel mit einer im Injektorgehäuse 30 angeordneten, nicht dargestellten Spindelmechanik in Wirkverbindung steht. Mittels dem am Ständer 12 angeordneten Schwenkkopf 11 ist das Injektorgehäuse 30, wie in Fig. 1 dargestellt, einerseits um eine Vertikalachse A in Pfeilrichtung P drehbar und andererseits um eine Horizontalachse A' entlang der Linie P' schwenkbar. Am vorderen Ende des Injektorgehäuses 30 ist ein Halterungskörper 20 angeordnet, in welchem eine auswechselbare Injektionsspritze 15 oder 15' eingesetzt ist.

In Fig. 2 ist teilweise im Schnitt der in Fig. 1 durch einen mit Y bezeichneten Kreis markierte Ausschnitt dargestellt und man erkennt den Halterungskörper 20, die Injektionsspritze 15 und ein Teilstück des Zylinderkörpers 14, welcher an dem einen Ende einen mit einer ringförmigen Nut 17 versehenen Flansch 18 sowie einen hinter dem Flansch liegenden, metallischen Ring 16 aufweist. An der Stirnseite des Flansches 18 sind mindestens zwei im Abstand zueinander angeordnete Spann-Nocken 19 vorgesehen.

Der Halterungskörper 20 besteht im wesentlichen aus einem Flansch 23, einem auf der einen Seite des Flansches 23 angeordneten Auflageteil 22 sowie einem auf der anderen Seite des Flansches 23 angeordneten Halterungsteil 27 für die Injektionsspritze. Der Halterungskörper 20 ist mit dem Flansch 23 in einer an der Gehäusestirnwand 32 vorgesehenen Oeffnung 33 gelagert und auf der anderen Seite durch zwei mit einem Befestigungsflansch 21 wirkverbundene Trägerelemente 34, 36 in dem Injektorgehäuse 30 befestigt. Der Flansch 23 ist mit einer in einer ringförmigen Nut 24 angeordneten Dichtung 25 in der Oeffnung 33 abgedichtet angeordnet. Die den Befestigungsflansch 21 durchdringenden Trägerelemente 34, 36 sind durch nicht näher dargestellte Klemmelemente 37, 38 mit dem Halterungskörper 20 wirkverbunden.

Das an dem Flansch 23 angeformte Halterungsteil 27 umfasst, wie in Fig. 2 dargestellt, eine im wesentlichen halbschalenförmig ausgebildete Wand 28 sowie ein parallel zum Flansch 23 angeordnetes Anlageteil 29, welches an der Innenseite 29' für die Zentrierung der Injektionsspritze ausgebildet ist.

Zwischen der Innenseite 29' des Anlageteils 29 und dem Flansch 23 sind zwei spiegelbildlich symmetrisch zueinander angeordnete und aus Federstahldraht geformte Federelemente 60, 62 angeordnet, wobei das Federelement 60 auf der den Spann-Nocken 19 zugewandten Seite ein angeformtes und in axialer Richtung federndes Teilstück 61 und das Federelement 62 ein entsprechendes Teilstück 63 aufweist. Die Injektionsspritze ist in das halbschalenförmig ausgebildete Halterungsteil 27 einsetzbar und wird durch die Rückstellkraft der Federteile 61, 63 gegen die Innenseite 29' des Anlageteils 29 gedrückt.

Weiterhin erkennt man in Fig. 2 einen ersten Tastbügel 55 einer Abtasteinrichtung 70. Der erste Tastbügel 55 besteht im wesentlichen aus einem Tastteil 56, einem Schwenkteil 57 und einem Betätigungsteil 58. Ein in Fig. 2 nicht sichtbarer zweiter Tastbügel 50 der Abtasteinrichtung 70 ist spiegelbildlich zu dem ersten Tastbügel 55 angeordnet und ausgebildet und umfasst ein Tastteil 51, ein Schwenkteil 52 sowie ein Betätigungsteil 53. Die beiden abgebogenen Tastteile 51 und 56 reichen in eingesetztem Zustand der Injektionsspritze in den Halterungskörper 20, in radialer Richtung mindestens bis zur Mitte des Zylinderkörpers 14 oder 14'. Die beiden Schwenkteile 52 und 57 durchdringen den Flansch 23 und sind in dem Flansch abgedichtet, schwenkbar gelagert. Die beiden Betätigungsteile 53 und 58 liegen auf einer Trägerplatte 40 und sind im wesentlichen durch eine mit Schrauben 43 an der Trägerplatte 40 befestigte Schiene 44 gehalten.

In Fig. 3 ist ein Teilstück der Injektionsspritze 15' dargestellt und man erkennt den Zylinderkörper 14', welcher im vorderen Bereich einen Flansch 18' und an der Flanschstirnseite zwei am Umfang gegenüberliegend angeordnete Spann-Nocken 19' sowie hinter dem Flansch 18' angeordnet einen Ring 16' aufweist.

Zur Unterscheidung des Zylinderquerschnitts der Injektionsspritze 15 gemäss Fig. 2 in bezug auf den Zylinderquerschnitt der Injektionsspritze 15' gemäss Fig. 3, ist der Flansch 18 des Zylinderkörpers 14 mit der Ringförmigen Nut 17 versehen.

In Fig. 4 und Fig. 5 sind in Draufsicht die beiden eingesetzten Spritzen-Varianten dargestellt und man erkennt das im Schnitt dargestellte Injektorgehäuse 30, die Seitenwände 31, 31' und die Stirnwand 32 des Injektorgehäuses sowie den mit dem Flansch 23 in der Oeffnung 33 gelagerten Halterungskörper 20. In Fig. 4 ist die eine bis zur Achse X-X reichende Hälfte mit eingesetzter Injektionsspritze 15 gemäss Fig. 2 und in Fig. 5 die andere, bis zur Achse X-X reichende Hälfte mit eingesetzter Injektionsspritze 15' gemäss Fig. 3 dargestellt. Der Halterungskörper 20 wird einerseits durch den in der Oeffnung 33 abgedichtet gelagerten Flansch 23 und andererseits durch die symmetrisch zur Achse X-X angeordneten Trägerelemente 34, 36 gehalten. Das Trägerelement 34 ist mittels der Klemmelemente 37 und das Trägerelement 36 mittels der Klemmelemente 38 in nicht näher dargestellter Weise an dem Befestigungsflansch 21 des Halterungskörpers 20 befestigt. Weiterhin erkennt man in den Figuren 2, 4 und 5 ein Teilstück eines Stössels 35, welcher auf der einen Seite den Halterungskörper 20 in nicht näher dargestellter Weise durchdringt und mit einem in dem Zylinderkörper 14 oder 14' der Injektionsspritze 15 oder 15' angeordneten Kolben 65 oder 65' in nicht dargestellter Weise wirkverbunden ist. Auf der anderen Seite ist der Stössel 35 mit der in dem Injektorgehäuse 30 angeordneten, nich dargestellten Spindelmechanik wirkverbunden, mittels welcher der Stössel 35 und somit auch der Kolben 65 oder 65' zum Injizieren einer Flüssigkeit in Pfeilrichtung 1 und nach dem Injektionsvorgang in Pfeilrichtung 1' bewegt wird.

Die in Fig. 4 und Fig. 5 in grösserem Massstab und in Draufsicht dargestellte Abtasteinrichtung 70 umfasst im wesentlichen die beiden spiegelbildlich symmetrisch zur Achse X-X angeordneten und den Flansch 23 des Halterungskörpers 20 durchdringenden Tastbügel 50, 55 sowie zwei auf der Trägerplatte 40 mit Schrauben 46, 47 befestigte Schalter 41 und 42.

Der aus Federstahldraht einstückig geformte erste Tastbügel 55 hat an dem einen Ende das Tastteil 56 und an dem anderen Ende das Betätigungsteil 58, wobei die beiden Teile 56 und 58 im wesentlichen durch das Schwenkteil 57 miteinander verbunden sind. Am Ende des Betätigungsteils 58 ist ein dem Druckknopf 42' des Schalters 42 zugeordnetes Kontaktelement 59 angeordnet und mit nicht dargestellten Mitteln befestigt. Der analog dem ersten Tastbügel 55 ausgebildete zweite Tastbügel 50 hat das Tastteil 51, das Betätigungsteil 53 sowie das die beiden Teile 51 und 53 miteinander verbindende Schwenkteil 52. Am Ende des Betätigungsteils 53 ist ein dem Druckknopf 41' des Schalters 41 zugeordnetes Kontaktelement 54 vorgesehen.

In Fig. 5 ist im Teilschnitt die Lagerung des zweiten Tastbügels 50 in dem Flansch 23 des Halterungskörpers 20 dargestellt und man erkennt eine den Flansch durchdringende Bohrung 26 in welcher zur beweglichen Lagerung des Schwenkteils 52 ein mit einer keilringförmigen Innenzentrierung versehenes Lagerteil 48 angeordnet ist. Zu beiden Seiten des Lagerteils 48 ist ein Dichtelement 49, 49' angeordnet. Das einzelne Dichtelement 49, 49' ist auf der einen Seite an der schrägen Fläche des Lagerteils 48 und auf der anderen Seite an der Umfangsfläche des Schwenkteils 52 durch eine Kleb- oder Vulkanisier-Verbindung befestigt.

Die Lagerung des Schwenkteils 57 von dem ersten Tastbügel 55 in dem Flansch 23 des Halterungskörpers 20 ist analog der voran beschriebenen Lagerung des Schwenkteils 52 ausgebildet.

Die Arbeitsweise der beschriebenen Abtasteinrichtung im Zusammenhang mit der in den Halterungskörper der Injektionsvorrichtung eingesetzten Injektionsspritze wird nachstehend beschrieben :

Einleitend sei darauf hingewiesen, dass die in dem Flansch 23 des Halterungskörpers 20 gelagerten Tastbügel 50 und 55, bei nicht eingesetzter Injektionsspritze, spiegelbildlich symmetrisch zueinander angeordnet sind, und dass die beiden Tastteile 51 und 56 einerseits und die beiden Betätigungsteile 53 und 58 andererseits, von der Achse X-X aus gesehen, in gleichem Abstand zueinander angeordnet sind. Der Abstand der beiden Tastteile 51 und 56 zueinander entspricht im dargestellten Ausführungsbeispiel dem Durchmesser der in dem Flansch 18 der Injektionsspritze 15 vorgesehenen ringförmigen Nut 17.

Ausgehend von dem in Fig. 4 dargestellten Ausführungsbeispiel, bei welchem die in dem Halterungskörper 20 eingesetzte Injektionsspritze 15 mit dem Spann-Nocken 19 an dem Teilstück 63 des Federelements 62 fest anliegt und mit dem Ring 16 gegen die Innenseite des Anlageteils 29 gedrückt wird, ist das Tastteil 56 des ersten Tastbügels 55 in der ringförmigen Nut 17 angeordnet und das Betätigungsteil 58 ist mit dem Kontaktelement 59 im Abstand zu dem Druckknopf 42' des Schalters 42 angeordnet. In dieser Stellung wird der Schalter 42 nicht betätigt und der Kolben 65 wird für den Injektionsvorgang mit einer ersten, vom Zylinderquerschnitt abhängigen Vorschubgeschwindigkeit gemäss Pfeilrichtung 1 in den Zylinderkörper der Injektionsspritze 15 geschoben. Bei dem in Fig. 5 dargestellten Ausführungsbeispiel, bei welchem die in dem Halterungskörper 20 eingesetzte Injektionsspritze 15' mit dem Spann-Nocken 19' an dem Teilstück 61 des Federelements 60 fest anliegt und mit dem Ring 16' gegen die Innenseite des Anlageteils 29 gedrückt wird, liegt das Tastteil 51 am äusseren Umfang des Flansches 18' fest an, so dass aufgrund der radialen Abstandsänderung der in dem Flansch 23 gelagerte Tastbügel 50 entsprechend ausgelenkt wird. Bei diesem Auslenk-Vorgang wird das Tastteil 51 radial nach aussen und das Betätigungsteil 53 radial nach innen bewegt, wodurch das am Betätigungsteil 53 angeordnete Kontaktelement 54 den Druckknopf 41' des Schalters 41 betätigt. Zur Regelung der vom Zylinderquerschnitt der Injektionsspritze 15' abhängigen Vorschubgeschwindigkeit der Stössel-Kolbeneinheit 35, 65' wird über eine im Steuer- und Bedienungspult 13 angeordnete Elektronik-Einrichtung ein entsprechendes Signal auf die Spindelmechanik übertragen.

Die beschriebene Abtasteinrichtung 70 in Verbindung mit den zugeordneten Schaltern 41, 42 eignet sich insbesondere zum Abtasten von zwei Injektionsspritzen mit unterschiedlichem Zylinderquerschnitt. Hierbei ist der eine Flansch 18 mit der im Durchmesser auf den Zylinderquerschnitt des Zylinderkörpers 14 bezogenen ringförmigen Nut 17 versehen (Fig. 4). Der andere, auf den Zylinderquerschnitt des Zylinderkörpers 14' bezogene Flansch 18' ist entweder mit einer im Durchmesser grösser bemessenen Ringnut, oder aber mit grösserem Flanschdurchmesser versehen (Fig. 5).

Bei Verwendung von entsprechend ausgebildeten Stufenschaltern, welche den beiden Betätigungselementen 53, 58 der Abtasteinrichtung 70 zugeordnet sind, besteht jedoch auch die Möglichkeit, unterschiedliche und im Flanschdurchmesser auf den jeweiligen Zylinderquerschnitt abgestimmte Injektionsspritzen in den Halterungskörper 20 einzusetzen. Die Regelung der vom Zylinderquerschnitt der Injektionsspritze abhängigen Vorschubgeschwindigkeit der Stössel-Kolbeneinheit wird über die bereits erwähnte Elektronik-Einrichtung erreicht, von welcher ein entsprechendes Signal auf die Spindelmechanik übertragen wird.

**Patentansprüche**

1. Injektionsvorrichtung (10) zur dosierten Abgabe einer Flüssigkeit, bestehend aus einem Injektorgehäuse (30), einem am vorderen Ende des Injektorgehäuses angeordneten Halterungskörper (20) und einer in dem Halterungskörper auswechselbar angeordneten Injektionsspritze (15, 15'), welche im wesentlichen einen Zylinderkörper (14, 14') mit einem Flansch (18, 18') an dem dem Halterungskörper zugewandten Ende und einen im Zylinderkörper geführten Kolben (65, 65') umfasst, der durch eine Kolbenstange (35) mit der motorisch angetriebenen Spindelwelle einer in dem Injektorgehäuse angeordneten Spindelmechanik wirkverbunden ist, dadurch gekennzeichnet, dass in dem Halterungskörper (20) mindestens ein Tastbügel (50, 55) auslenkbar gelagert ist, welcher auf der einen Seite ein den Flansch (18, 18') des Zylinderkörpers (14, 14') kontaktierendes Tastteil (51, 56) und auf der anderen Seite in Injektorgehäuse ein Betätigungsteil (53, 58) aufweist, welchem mindestens ein Schalter (41, 42) zugeordnet ist, der gewährleistet, dass bei ausgelenktem Tastbügel (50, 55) zur Einstellung der Vorschubgeschwindigkeit des mit der Kolbenstange (35) wirkverbundenen Kolbens (65, 65') ein vom Zylinderquerschnitt abhängiges Signal auf die Spindelmechanik übertragen wird.

2. Injektionsvorrichtung nach Anspruch 1, dadurch gekennzeichnet, dass in dem Halterungskörper (20) zwei spiegelbildlich symmetrisch zueinander angeordnete Tastbügel (50, 55) angeordnet und je mit einem Schwenkteil (52, 57) in einem Flansch (23) des Halterungskörpers abgedichtet, schwenkbar gelagert sind.

3. Injektionsvorrichtung nach Anspruch 1, dadurch gekennzeichnet, dass der Flansch (18, 18') des Zylinderkörpers (14, 14') einen in Abhängigkeit vom Zylinderquerschnitt ausgebildeten äusseren Durchmesser aufweist.

4. Injektionsvorrichtung nach den Ansprüchen 1 und 3, dadurch gekennzeichnet, dass zur Führung des Tastteils (51, 56) am Flansch (18, 18') eine im äusseren Durchmesser vom Zylinderquerschnitt abhängige ringförmige Nut (17) vorgesehen ist, und dass das in der ringförmigen Nut (17) geführte Tastteil (51, 56) in radialer Richtung mindestens bis zur Mitte des in den Halterungskörper eingesetzten Zylinderkörpers (14, 14') reicht.

## Claims

1. Injection device (10) for dispensing controlled amounts of fluid, comprising an injector housing (30), a support body (20) arranged at the front end of the injector housing, and an injection nozzle (15, 15') replaceably arranged in the support body and which substantially comprises a cylinder body (14, 14') with a flange (18, 18') on the end facing the support body, and a piston (65, 65') guided in the cylinder body, which piston is operatively connected by means of a piston rod (35) to the motor driven spindle shaft of a spindle mechanism arranged in the injector housing, characterised in that in the support body (20) there is deflectably mounted at least one sensing yoke (50, 55) which has on one side a sensing part (51, 56) contacting the flange (18, 18') of the cylinder body (14, 14'), and on the other side in the injector housing an actuation part (53, 58) with which there is associated at least one switch (41, 42), which provides that when the sensing yoke (50, 55) is deflected, a signal dependent on the cylinder cross-section is transmitted to the spindle mechanism to adjust the thrust speed of the piston (65, 65') operatively connected to the piston rod (35).

2. Injection device according to claim 1, characterised in that in the support body (20) there are arranged two sensing yokes (50, 55) which are arranged symmetrically with respect to one another and in mirror-image fashion and are mounted swivellably by respective swivel portions (52, 57), sealed in a flange (23) of the support body.

3. Injection device according to claim 1, characterised in that the flange (18, 18') of the cylinder body (14, 14') has an outer diameter designed in dependence on the cylinder cross-section.

4. Injection device according to claims 1 and 3, characterised in that in order to guide the sensing part (51, 56) there is provided on the flange (18, 18') an annular groove (17) in the outer diameter dependent on the cylinder cross-section, and in that the sensing part (51, 56) guided in the annular groove (17) in the radial direction reaches at least to the centre of the cylinder body (14, 14') inserted into the support body.

## Revendications

1. Dispositif d'injection (10) de quantités dosées d'un fluide, comprenant un boîtier d'injection (30), un corps de support (20) prévu à l'extrémité avant du boîtier d'injection et une seringue d'injection (15, 15') montée de façon interchangeable dans le corps de support, qui se compose essentiellement d'un corps cylindrique (14, 14') avec une bride (18, 18') à l'extrémité tournée vers le corps de support ainsi qu'un piston (60, 65') guidé dans le corps de cylindre, et qui est relié de façon active par une tige de piston (35) à un axe de broche entraîné par un moteur appartenant à un mécanisme de broche logé dans le boîtier d'injection, caractérisé en ce que dans le corps de support (20), il est prévu au moins un étrier de détection (50, 55) monté de façon à pouvoir dévier, et qui comporte d'un côté une partie de détection (51, 56) touchant la bride (18, 18') du corps de cylindre (14, 14') et de l'autre côté une partie de manœuvre (53, 58) dans le boîtier d'injection, et à laquelle est associé au moins un commutateur (41, 42) qui, lorsque l'étrier de détection (50, 55) est dévié, assure la transmission d'un signal dépendant de la section de cylindre vers le mécanisme de broche pour régler la vitesse d'avance du piston (65, 65') relié de façon active à la tige de piston.

2. Dispositif d'injection selon la revendication 1, caractérisé en ce que dans le corps de support (20) se trouvent deux étriers de détection (50, 55) montés l'un par rapport à l'autre de façon symétrique par rapport à un plan, et de façon étanche dans une bride (23) du corps de support par l'intermédiaire d'une partie de basculement (52, 57).

3. Dispositif d'injection selon la revendication 1, caractérisé en ce que la bride (18, 18') du corps de cylindre (14, 14') comporte un diamètre extérieur réalisé en fonction de la section de cylindre.

4. Dispositif d'injection selon l'une des revendications 1 et 3, caractérisé en ce que pour guider la partie de détection (51, 56) sur la bride (18, 18'), il est prévu une rainure (17), annulaire dépendant du diamètre extérieur de la section de cylindre, et en ce que la partie de détection (51, 56) guidée dans la rainure annulaire (17) arrive dans la direction radiale au moins jusqu'au milieu du corps de cylindre (14, 14') placé dans le corps de support.

**0 080 037**

FIG. 1

FIG. 2

FIG. 3

FIG. 4

FIG. 5